# EUROPEAN PATENT APPLICATION

(11) **EP 2 025 326 A1**
(43) Date of publication of application: **18.02.2009**
(21) Application number: 08013978.5
(22) Date of filing: 05.08.2008
(51) Int. Cl.: A61K 8/44, A61Q 19/10

(54) **A cosmetic liquid**

(30) Priority: 10.08.2007 JP 2007209163
(71) Applicant: Sansho Cosme Inc., Sumiyosi-ku Osaka-shi Osaka (JP)
(72) Inventor: Katsuhito, Hatanka, Osaka (JP); Ohira, Hitomi, Osaka (JP)
(74) Representative: Graalfs, Edo

(57) **Abstract**

The present invention relates to cosmetic liquid having pH of 9 to 13 and containing an organic amine compound.

## Description

### BACKGROUND OF THE INVENTION

### Field of the invention

The present invention relates.to a cosmetic liquid.

### Prior Art

The wording "cosmetic liquid" referred to here is adopted as a concept including a lotion and an essence.

The lotion is, for example, provided for the purpose of supplying moisture and any moisturizing elements to skin to keep skin fresh, youthful, and dewy. The lotion is ordinarily transparent or translucent and in liquid state.

The lotion is, ordinarily, slightly acidic, and moreover has been provided in such three kinds as an acidic lotion, an alkaline lotion, and a neutral lotion. The slight acidic lotion is ordinarily applied to skin as being adapted to pH of skin and not having a particular stimulus thereto. Most of the acidic lotion is applied to make healthy pH of skin which ordinarily tends Lo become alkaline from Spring to Summer seasons. And alkaline lotion is applied to provide moisture to skin.

These conventional types of lotions cause users to less feel moist and do not show an excellent effect of making soft skin.

### SUMMARY OF THE INVENTION

In view of the above circumstances, the Inventor zealously studied and achieved the cosmetic liquid according to the present invention which is characterized in that pH is 9 through 13.

### DETAILED DESCRIPTION OF THE INVENTION

The conventional lotion (even the alkaline lotion) has a pH of 7 to 8 at the most and no more. This is because skin is slightly acidic, and for the purpose of eliminating a stimulus to skin the lotion essentially needs to have the same degrees of pH as those of skin. In fact, the alkaline lotion has a stimulus to skin.

Under the circumstance, the inventor studied the rising of pH of the lotion while bearing the lotion's stimulus to the inventor's skin and finally achieved the present invention. Resultantly, the present invention is to be said as an ultra-alkali cosmetic liquid which is quite different from the conventional alkaline lotion.

The pH of the cosmetic liquid according to the present invention is 9 to 13. Hitherto there has never been a product of such high pH values. No one had any ideas of such product in consideration of stimuli to skin. The product does in fact have a stimulus to skin.

Among the pH values 9 to 13, the pH 10 to 12 is most preferable from balancing between the stimuli and the effects of the cosmetic liquid.

The cosmetic liquid does, naturally, contain the ordinary constituents for realizing the ordinary cosmetic liquid, however, in which there are added the materials for rising the pH values.

The ordinary constituents may be water, alcohols, humectant, softening agent, solubilizing agent, viscosity bodying agent, perfume, antiseptic agent, coloring agent, anti-fading agent, and/or, drugs, and are not particularly defined.

The constituents for rising the pH values may employ any materials having high pH values, and preferably, organic alkaline matters, in consideration of skin. In detail, those preferable may be amine, such as monoethanolamine, diethanolamine, triethanolamine, etc. Inorganic materials may be usable naturally, but, organic ones are superior in respect of the properties of stimulus, etc.

More preferable may be basic amino acid. Amino acid has both of amino group and carboxyl group and is basic when having many amino groups, for example, as lysine, hydroxylysine, arginine, etc.

Essential amino acid needed for human bodies when applied shows such an excellent effect as not only merely rising the pH values but also providing nourishment to human bodies.

### PREFERRED EMBODIMENTS OF THE INVENTION

Next, the present invention will be further detailed with referring to the specific Examples.

### Example 1

### Constituents

Water and hot spring water, adopted as main constituents, are mixed with a constituent for rising the pH values, a humectant, and an antiseptic agent to prepare the Example. Specific constituents are as shown in Table 1.

In the Table 1, BG is 1-3, dibutylene glycol, and PE is phenoxyethanol.

Among the constituents, hot spring water (taken from Izumo Yumura hot spring, etc) is allowed, with this naming, as a constituent for cosmetics. Hot spring water (from a particular hot spring) as it is has been employed here.

Glycerin is mixed for providing the humectant effect, and BG and PE mixed as antiseptic agents.

Comparative examples to those of the present invention are also shown in Table 1.

**(TABLE 1)**

| | | Ex.1 | Ex2 | Ex.3 | Com.1 | Com.2 | Com.3 | Com.4 |
|---|---|---|---|---|---|---|---|---|
| | Water | 49.00 | 48.50 | 46.00 | 49.49 | 49.00 | 49.00 | 48.00 |
| | Hot spring water | 40.00 | 40.00 | 40.00 | 40.00 | 40.00 | 40.00 | 40.00 |
| Humectant | Glycerin | 5.00 | 5.00 | 5.00 | 5.00 | 5.00 | 5.00 | 5.00 |
| Anti-septic agent | BG | 5.00 | 5.00 | 5.00 | 5.00 | 5.00 | 5.00 | 5.00 |
| | PE | 0.50 | 0.50 | 0.50 | 0.50 | 0.50 | 0.50 | 0.50 |
| pH adjustment | Argynine | 0.50 | - | 0.50 | 0.01 | - | - | 0.50 |
| | Tri-othanole amine | - | 1.00 | 3.00 | - | - | - | - |
| | Potassium hydroxide | - | - | - | - | - | - | 1.00 |
| Amino acid | Threonine | - | - | - | - | 0.50 | - | - |
| | Proline | - | - | - | - | - | 0.50 | - |
| Total | | 100.00 | 100.00 | 100.00 | 100.00 | 100.00 | 100.00 | 100.00 |
| pH | | 10.65 | 9.85 | 11.00 | 7.43 | 7.80 | 6.80 | 13.19 |
| Effects | Softened | ○ | ○ | ○ | × | × | × | ○ |
| | Moist | ○ | ○ | ○ | Δ | Δ | Δ | ○ |
| | Stimuli | ○ | ○ | ○ | ○ | ○ | ○ | × |
| | Dirt-removing | ○ | Δ | ○ | Δ | × | × | ○ |

| | | | | | | | | |
|---|---|---|---|---|---|---|---|---|
| Numerical values: weight per cent (%) | | | | | | | | |

Effects shown in Table 1 are opinions expressed by ten women who tried the cosmetic liquids of the Examples and Comparative examples on their faces upon cleansing and after washing faces.

The signs expressed at the Effects have specific meanings as follows. ○ at "Softened" means that skin becomes soft sufficiently, and × means that there is almost no such effect. ○ at "Moist" means that skin becomes fully moist, and Δ moist slightly. ○ at "Stimuli" means that there is almost no stimulus, and × means there are definitely stimuli. ○ at "Dirt removing' means that dirt is removed well, Δ removed moderately, and × dirt not so removed.

All the Examples showed excellent results while the Comparative examples were all problematic. Comparative examples 1 to 3 had low pH values not within the extent of pH values of the present invention and were poor in the skin-softening efficiency. Moreover, the Comparative example 4 was too high in pH value and had a strong stimulus.

### Effect of the invention

The cosmetic liquid according to the present invention has the Following advantages.
(1) Since the cosmetic liquid has very high pH values, it is stimulative to skin, while it weakens binding of protein, so that it can be readily adsorbed by skin which is slightly acidic.
(2) Skin is made soft thanks to becoming alkaline.
(3) Skin becoming alkaline has an increased amount of moisture to thereby be moist, eliminating feel of being dry and rough to the touch.
(4) Since sebum and dirt are well removed, the cosmetic liquid can be us able as a wiping lotion.

## Claims

1. A cosmetic liquid having pH of 9 to 13.

2. A cosmetic liquid as set forth in Claim 1 wherein the cosmetic liquid contains an organic amine compound.

3. A cosmetic liquid as set forth in Claim 2 wherein the organic amine compound is basic amino acid.
